# EUROPÄISCHE PATENTSCHRIFT

(11) **EP 0 586 961 B1**
(45) Veröffentlichungstag und Bekanntmachung des Hinweises auf die Patenterteilung: **26.11.1997**
(21) Anmeldenummer: 93113546.1
(22) Anmeldetag: 25.08.1993
(51) Int. Cl.: A61K 7/42, A61K 7/00, A61K 31/415

(54) **Kosmetische und dermatologische Formulierungen mit einem wirksamen Gehalt an cis-Urocaninsäure**
Cosmetic and dermatologic formulations containing an effective amount of cis urocanic acid
Formulations cosmétiques et dermatologiques contenant une teneur effective d'acide cis-urocanique

(30) Priorität: 09.09.1992 DE 4230076
(43) Veröffentlichungstag der Anmeldung: 16.03.1994
(73) Patentinhaber: Beiersdorf Aktiengesellschaft, 20245 Hamburg (DE)
(72) Erfinder: Stäb, Franz, Dr., D-21379 Echem (DE); Sauermann, Gerhard, Dr., D-24649 Wiemersdorf (DE); Uhlmann, Beate, Dr., D-22453 Hamburg (DE)

(56) Entgegenhaltungen:
- EP-A- 0 467 116
- WO-A-86/05783
- US-A- 4 181 804
- S.T.N., Datenbank Lifferant, KARLSRUHE, DE Datenbank Chemical Abstracts, Band 92, n 185715 * zusammenfassung *
- S.T.N., Datenbank Lifferant, KARLSRUHE, DE Datenbank Chemical Abstracts, Band 116, n 230836 * zusammenfassung *

## Beschreibung

Die vorliegende Erfindung betrifft Lichtschutzmittel, insbesondere hautpflegende kosmetische und dermatologische Lichtschutzmittel, sowie kosmetische und dermatologische Zubereitungen, solche Lichtschutzmittel enthaltend. Insbesondere betrifft die Erfindung auch Hautreinigungsprodukte, solche Lichtschutzmittel enthaltend.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)) nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzlich Antioxidantien und/oder Radikalfänger einverleibt werden.

Die teilweise vorstehend genannten Verbindungen, welche als Lichtschutzmittel für kosmetische und dermatologische Lichtschutzformulierungen eingesetzt werden, zeichnen sich an sich durch gute Lichtschutzwirkung aus. Sie haben jedoch den Nachteil, daß es bisweilen schwierig ist, sie in befriedigender Weise solchen Formulierungen einzuverleiben. Ferner sind diese Verbindungen reine UV-Absorber und als Radikalfänger ungeeignet.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Es ist auch bekannt, trans-Urocaninsäure (auch trans-Urocansäure, E-Urocaninsäure, E-Urocansäure, trans-(4-Imidazolyl)acrylsäure oder E-4-Imidazolylacrylsäure genannt) als Lichtschutzmittel einzusetzen.

Beispiele finden sich in den japanischen Offenlegungsschriften JP-Kokai-Sho-54/027562, JP-Kokai-Sho-63/051318 und JP-Kokai-Sho-56/063965, bzw. in den dazugehörigen Auslegeschriften.

trans-Urocaninsäure ist durch folgende Strukturformel gekennzeichnet:

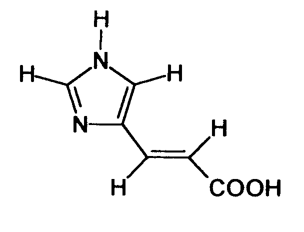

In Chemical Abstracts 92, 185715c wird die Lichtschutzwirkung der geometrischen Isomere der Urocaninsäure und ihrer Natriumsalze beschrieben.

Es war indes überraschend und für den Fachmann nicht vorauszusehen, daß die Verwendung einer wirksamen Menge an cis-Urocaninsäure als Antioxidans, zur Herstellung von Lichtschutzformulierungen für kosmetische und/oder dermatologische Zwecke den Nachteilen des Standes der Technik abhelfen würde.

cis-Urocaninsäure (auch cis-Urocansäure, Z-Urocaninsäure, Z-Urocansäure, trans-(4-Imidazolyl)acrylsäure oder Z-4-Imidazolylacrylsäure genannt) ist durch folgende Strukturformel gekennzeichnet:

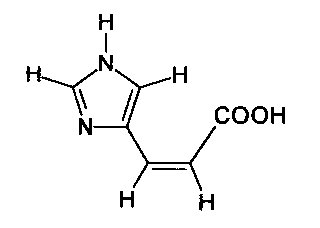

Sie hat die Summenformel C₆H₆N₂O₂ und die Molekularmasse 138,12. cis-Urocaninsäure entsteht beispielsweise durch UV-Bestrahlung des trans-Isomeren, weiches in der menschlichen Haut und auch im Schweiß vorkommt.

Es war nicht vorherzusehen gewesen, daß die cis-Urocaninsäure oder kosmetische oder dermatologischen Zubereitungen mit einem wirksamen Gehalt an cis-Urocaninsäure
- besser als Antioxidans wirken
- besser als Radikalfänger wirken
- besser die Bindung von schädlichen Photoprodukten an Lipide, DNS und Proteine verhindern
würden als die Zubereitungen des Standes der Technik. Ferner war nicht vorherzusehen gewesen, daß die cis-Urocaninsäure oder die erfindungsgemäßen kosmetischen oder dermatolgischen Zubereitungen mit einem wirksamen Gehalt an cis-Urocaninsäure
- für die Anwendung genügend hohe Stabilität aufweisen
- zu hautverträglichen Produkten führen
- nicht in die hauteigene Mikroorganismenflora eingreifen
- sogar in waschaktiven Zubereitungen wie Shampoos und Duschzubereitungen usw. verfügbar und wirksam sein
- die Hautfeuchtigkeit steigern und
- das Auswaschen der hauteigenen Urocaninsäure kompensieren
würden.

Ferner war überraschend, daß cis-Urocaninsäure auch als Antioxidans bei auf der menschlichen Haut relevanten photochemischen Oxidationsprozessen wirksam sein würde. Die antioxidative physiologische Wirkung der cis-Urocaninsäure - welche vermutlich auf deren Eigenschaff als Radikalfänger zurückzuführen ist - war bisher nicht bekannt.

Zwar ist aus der DE-OS 41 21 030 bekannt, cis-Urocaninsäure in dermatologische Formulierungen zu geben, welche mancherlei Wirkung zeitigen, darunter antipsoriatische, antiallergische und dergleichen. Dieses Dokument legt jedoch an keiner Stelle die vorteilhaften Eigenschaften der cis-Urocaninsäure als Antioxidans und Radikalfänger auch nur nahe.

Erfindungsgemäß können kosmetische und/oder dermatologische Formulierungen wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0,01 Gew.-% bis 10 Gew.-%, insbesondere aber 0,1 Gew.-% bis 6 Gew.-%, bezogen auf das Gesamtgewicht des Mittels, an cis-Urocaninsäure.

Allerdings wird auf die entsprechenden Verordnungen der einzelnen Staaten hingewiesen, welche im Einzelfalle Höchstwerte für Wirkstoffkonzentrationen festsetzen. In Deutschland ist zum gegenwärtigen Zeitpunkt die Höchstkonzentration an Urocaninsäure (cis- und trans-Isomer zusammengenommen) auf 2,0 Gew.%, bezogen auf das Gesamtgewicht der Zusammensetzung, beschränkt.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zuberertungen, die in der Form eines Sonnenschutzmittels vorliegen. Vorzugsweise enthalten diese zusätzlich mindestens einen UVA-Filter und/oder mindestens einen weiteren UVB-Filter und/oder mindestens ein anorganisches Pigment.

Erfindungsgemäß können kosmetische und dermatologische Zubereitungen zum Schutze der Haut vor UV-Strahlen in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, einen festen Stift oder auch ein Aerosol darstellen.

Es ist auch möglich und vorteilhaft im Sinne der vorliegenden Erfindung, cis-Urocaninsäure in wäßrige Systeme bzw. Tensidzubereitungen zur Reinigung der Haut und der Haare einzufügen.

Erfindungsgemäß können kosmetische und dermatologische Zubereitungen kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Emulsionen gemäß der Erfindung z.B. in Form einer Sonnenschutzcreme oder einer Sonnenschutzmilch sind bevorzugt und enthalten z.B. die genannten Fette, Öle, Wachse und anderen Fettkörper, sowie Wasser und einen Emulgator, wie er üblicherweise für einen solchen Typ der Formulierung verwendet wird.

Gele enthalten gemäß der Erfindung üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Feste Stifte enthalten gemäß der Erfindung z.B. natürliche oder synthetische Wachse, Fettalkohole oder Fettsäureester. Bevorzugt werden Lippenpflegestifte.

Als Treibmittel für erfindungsgemäße, aus Aerosolbehältern versprühbare kosmetische Desodorantien sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die kosmetischen oder dermatologischen Zubereitungen zum Schutze der Haut enthalten Verbindungen der Formel I z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-% bezogen auf das Gesamtgewicht der Zubereitungen.

Bevorzugt können sie außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester; Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5- triazin.

Als wasserlösliche Substanzen sind z.B. zu nennen:
- Salze der 2-Phenylbenzimidazol-5-sulfonsäure wie ihr Natrium, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und ihre Salze.

Die Liste der genannten UVB-Filter, die in Kombination mit den Verbindungen der allgemeinen Formel I verwendet werden können, soll selbstverständlich nicht limitierend sein.

Gegenstand der Erfindung ist auch die Kombination eines erfindungsgemäßen UVA-Filters mit einem UVB-Filter bzw. eine erfindungsgemäßes kosmetische oder dermatologische Zubereitung, welche auch einen UVB-Filter enthält.

Es kann auch von Vorteil sein, cis-Urocaninsäure mit UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.-Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Kosmetische und dermatologische Zubereitungen mit einem wirksamen Gehalt an cis-Urocaninsäure können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und Pigment bzw. Zubereitungen, die diese Kombination enthalten, sind Gegenstand der Erfindung. Es können die für die vorstehenden Kombinationen genannten Mengen verwendet werden.

Bei kosmetischen und dermatologischen Zubereitungen zum Schutze der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel. Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektrolyte, Substanzen gegen das Fetten der Haare.

Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, mindestens eine Verbindung der allgemeinen Formel I im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetischen oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie cis-Urocaninsäure in wirksamer Konzentration. Die Menge der verwendeten Polymeren liegt z.B. zwischen 0,1 und 10 Gew.-%. bevorzugt zwischen 0,1 und 3 Gew.-%.

Kosmetische Zubereitungen zur Behandlung und Pflege der Haare können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Kosmetische Zubereitungen zur Behandlung und Pflege der Haare können als Gele vorliegen, die neben einem wirksamen Gehalt an cis-Urocaninsäure und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge der cis-Urocaninsäure in einem für die Haare bestimmten Mittel 0,05 Gew.-% bis 10 Gew.%, insbesondere 0,5 Gew.-% bis 5 Gew.-%, bezogen auf das Gesamtgewicht des Mittels.

Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgenden Beispiele sollen die vorliegende Erfindung verdeutlichen, ohne sie einzuschränken. UCS bedeutet dabei stets cis-Urocaninsäure.

### Beispiel 1

| Wäßrige Zubereitung (Gesichtswasser) | |
|---|---|
| | Gew.-% |
| PEG-40-hydrogenated Castor Oil | 0,811 |
| Dipropylenglycol | 2,534 |
| PEG-8 | 1,521 |
| Na₃EDTA | 0,253 |
| Polymer JR 125 | 0,025 |
| UCS | 0,750 |
| Wasser VES | ad 100,000 |

### Beispiel 2

| Wäßrige Zusammensetzung | |
|---|---|
| | Gew.-% |
| Polyfettsäureester (Cetiol HE) | 16,000 |
| PPG-3-Myristylether (Witconol APM) | 1,000 |
| Propylenglycol | 3,000 |
| Glycerin | 40,000 |
| UCS | 0,500 |
| Wasser VES | ad 100,000 |

### Beispiel 3

| Hydrogel (Polyacrylatgel) | |
|---|---|
| | Gew.-% |
| Acrylsäurepolymerisat (Carbopol 934) | 1,000 |
| Tris(hydroxymethylamino)methan (Tris) | 1,000 |
| Glycerin | 2,000 |
| Propylenglycol | 2,000 |
| UCS | 0,050 |
| Wasser VES | ad 100,000 |

### Beispiel 4

| Hochwasserhaltige Zubereitung (sehr weich) | |
|---|---|
| | Gew.-% |
| Ceteareth (Cremophor A 25) | 0,100 |
| Cetearyl Alcohol (Lanette O) | 0,400 |
| Vaseline, DAB 9 | 12,500 |
| Mineralöl, DAB 9 | 11,000 |
| Ceteareth-6-stearylalkohol (Cremophor A6) | 6,000 |
| UCS | 0,020 |
| Wasser VES | ad 100,000 |

### Beispiel 5

| Hochwasserhaltige Zubereitung (weich) | |
|---|---|
| | Gew.-% |
| Ceteareth-25 (Cremophor A25) | 1,500 |
| Cetearyl Alcohol (Lanette O) | 8,500 |
| UCS | 0,250 |
| Wasser VES | ad 100,000 |

### Beispiel 6

| Hochwasserhaltige Zubereitung (weich) | |
|---|---|
| | Gew.-% |
| Ceteareth-25 (Cremophor A25) | 2,000 |
| Cetearylalcohol (Lanette O) | 8,000 |
| Vaseline, DAB 9 | 10,000 |
| Mineralöl, DAB 9 | 10,000 |
| UCS | 0,100 |
| Wasser VES | ad 100,000 |

### Beispiel 7

| Hochwasserhaltige Zubereitung (mittelfest) | |
|---|---|
| | Gew.-% |
| Ceteareth-25 | 3,000 |
| Cetearyl Alcohol (Lanette O) | 17,000 |
| UCS | 0,175 |
| Wasser VES | ad 100,000 |

### Beispiel 8

| Dünnflüssige Lotion | |
|---|---|
| | Gew.-% |
| Ceteareth-25 (Cremophor A25) | 1,000 |
| Ceteareth-6-stearylalcohol (Cremophor A6) | 1,000 |
| Glycerin-mono-distearat (Tegin normal) | 2,000 |
| Cetylalcohol | 1,000 |
| Isopropylmyristat | 1,450 |
| Glycerin | 1,000 |
| Polyvinylpyrrolidon | 0,500 |
| UCS | 0,125 |
| Wasser VES | ad 100,000 |

### Beispiel 9

| Dickflüssige Lotion | |
|---|---|
| | Gew.-% |
| Ceteareth 25 (Cremophor A25) | 2,000 |
| Cetearyl Alcohol (Lanette O) | 3,000 |
| Mineralöl, DAB 9 | 5,000 |
| Propylenglycol | 3,000 |
| Polyvinylpyrrolidon | 0,500 |
| UCS | 0,300 |
| Wasser VES | ad 100,000 |

### Beispiel 10

| W/O-Crème | |
|---|---|
| | Gew.-% |
| Glycerinsorbitanfettsäureester (Arlacel 481) | 6,000 |
| Mikrokristallines Wachs (Lunacera M) | 1,000 |
| Neutralöl | 3,000 |
| Paraffinöl | 19,000 |
| Magnesiumstearat | 1,000 |
| Propylenglycol | 3,700 |
| Magnesiumsulfat (MgSO₄*7 H₂O) | 0,700 |
| UCS | 1,000 |
| Wasser VES | ad 100,000 |

### Beispiel 11

| W/O-Emulsion | |
|---|---|
| | Gew.-% |
| Polyoxyethylen-Glycerin-Sorbitan-Fettsäureester (Arlacel 988) | 3,600 |
| Polyoxyethylen-Fettsäureester (Arlacel 989) | 1,400 |
| Cetearyl Alcohol (Lanette O) | 2,000 |
| Mineralöl, DAB 9 | 25,000 |
| Paraben-Mischung | nach Belieben |
| Magnesiumsulfat (MgSO₄*7 H₂O) | 0,700 |
| UCS | 1,250 |
| Wasser VES | ad 100,000 |

### Beispiel 12

| W/O-Lotion | |
|---|---|
| | Gew.-% |
| Glycerinsorbitanfettsäureester (Arlacel 481) | 1,300 |
| Polyoxyethylen-Fettsäureester (Arlacel 989) | 3,700 |
| Neutralöl (Miglyol) | 6,000 |
| Paraffinöl, DAB 9 | 14,000 |
| Propylenglycol | 3,800 |
| Magnesiumsulfat (MgSO₄*7 H₂O) | 0,700 |
| UCS | 0,060 |
| Wasser VES | ad 100,000 |

### Beispiel 13

| O/W-Emulsion | |
|---|---|
| | Gew.-% |
| PEG-100-Stearate (Arlacel 165) | 5,000 |
| Cetearyl Alcohol (Lanette O) | 3,000 |
| Mineralöl, DAB 9 | 25,000 |
| Paraben-Mischung | nach Belieben |
| UCS | 0,325 |
| Wasser VES | ad 100,000 |

### Beispiel 14

| O/W-Emulsion | |
|---|---|
| | Gew.-% |
| Polysorbate-60 (Tween 60) | 3,000 |
| Sorbitan Stearate (Arlacel 60) | 2,000 |
| Cetearyl Alcohol (Lanette O) | 3,000 |
| Mineralöl, DAB 9 | 25,000 |
| Paraben-Mischung | nach Belieben |
| UCS | 0,035 |
| Wasser VES | ad 100,000 |

### Beispiel 15

| Kationenaktive Emulsion | |
|---|---|
| | Gew.-% |
| Distearyldimethylammoniumchlorid (Genamin DS AC) | 5,000 |
| Vaseline, DAB 9 | 5,000 |
| Isopropylpalmitat | 2,000 |
| Cetylalcohol | 1,000 |
| Siliconöl | 0,100 |
| Propylparaben | 0,100 |
| Methylparaben | 0,100 |
| Glycerin | 4,000 |
| UCS | 0,090 |
| Wasser VES | ad 100,000 |

### Beispiel 16

| Ionische Emulsion | |
|---|---|
| | Gew.-% |
| Natrium Cetearylsulfat (Emulgade F) | 6,000 |
| Mineralöl, DAB 9 | 25,000 |
| Paraben-Mischung | nach Belieben |
| UCS | 0,450 |
| Wasser VES | ad 100,000 |

### Beispiel 17

| Ionische O/W-Emulsion | |
|---|---|
| | Gew.-% |
| Stearinsäure | 5,000 |
| Cetearyl Alcohol (Lanette O) | 3,000 |
| Mineralöl, DAB 9 | 25,000 |
| Paraben-Mischung | nach Belieben |
| Triethanolamin | 1,000 |
| UCS | 0,080 |
| Wasser VES | ad 100,000 |

### Beispiel 18

| Sonnenöl | |
|---|---|
| UCS | 30.0 g |
| 3-(4'-Methylbenzyliden)campher, ("Eusolex 6300", Merck) | 60,0 g |
| Myristylalkohol, polyoxypropyliert mit 3 Mol Propylenoxid ("Witconol APM", Witco) | 608,0 g |
| C₁₂-C₁₅-Alkoholbenzoat ("Finsolv TN", Witco) | 152,0 g |
| Glycerinmonococoat, polyoxyethyliert mit 7 mol Ethylenoxid ("Cetiol HE", Henkel KGaA | 100,0 g |
| Ethanol | 65,0 g |
| 2-Octadodecanol | 20,0 g |
| Parfüm, Korrigentien, Additive, Antioxidantien, Stabilisatoren | nach Belieben |

Die Bestandteile des Sonnenöls werden miteinander vermischt und dabei gegebenenfalls auf 40 bis 50°C zur Homogenisierung erwärmt.

### Beispiel 19

| Sonnengel | |
|---|---|
| UCS | 18,0 g |
| 2,4,6-Trianilino-(p-carbo-2'-ethylhexyl-1'-oxy)-1,3,5-triazin ("Uvinul" T-150, BASF) | 25,0 g |
| Isopropylmyristat | 189,0 g |
| C₁₂-C₁₅-Alkoholbenzoat ("Finsolv TN", Witco) | 76,0 g |
| Myristylalkohol, polyoxypropyliert mit 3 Mol Propylenoxid ("Witconol APM", Witco) | 304,0 g |
| Capryl-/Caprinsäuretriglycerid ("Miglyol-Neutralöl", Dynamit-Nobel) | 195,0 g |
| "Bentone-38", Kronos-Titan | 150,0 g |
| Propylencarbonat | 20,0 g |
| Ethanol | 23,0 g |
| Parfüm, Korrigentien, Additive, Antioxidantien, Stabilisatoren | nach Belieben |

Mit den genannten Bestandteilen wird in üblicher Weise ein Sonnengel hergestellt.

### Beispiel 20

| Hydrogel | |
|---|---|
| UCS | 15,0 g |
| 2-Phenylbenzimidazol-5-sulfonsäure, ("Eusolex 232", Merck) | 27,0 g |
| Allantoin | 2,0 g |
| Sorbit fl. ("Karion F", Merck) | 22,0 g |
| "Carbopol 934", B.F. Goodrich | 15,0 g |
| Tris (hydroxymethyl)aminomethan | 27,0 g |
| Propylenglykol | 10,0 g |
| Ethanol | 300,0 g |
| Wasser | 582,0 g |
| Parfüm, Korrigentien, Additive, Antioxidantien, Stabilisatoren | nach Belieben |

Mit den genannten Bestandteilen wird in üblicher Weise ein Hydrogel hergestellt.

### Beispiel 21

| Öl-in-Wasser-Emulsion (Sonnencreme) | |
|---|---|
| UCS | 20,0 g |
| 2-Phenylbenzimidazol-5-sulfonsäure ("Eusolex 232", Merck) | 32,0 g |
| Stearylalkohol, der mit 2 Mol Ethylenoxid oxyethyliert ist ("Brij 72", ICI) | 30,0 g |
| Stearylalkohol, der mit 21 Mol Ethylenoxid oxyethyliert ist ("Brij 721", ICI) | 20,0 g |
| Cetylstearylalkohol | 25,0 g |
| Myristylalkohol, polyoxypropyliert mit 3 Mol Propylenoxid ("Witconol APM", Witco) | 64,0 g |
| C₁₂-C₁₅-Alkoholbenzoat ("Finsolv TN", Witco) | 16,0 g |
| Propylenglykol | 35,0 g |
| Tris(hydroxymethyl)aminomethan | 14,0 g |
| Wasser | 744,0 g |
| Parfüm, Korrigentien, Additive, Antioxidantien, Stabilisatoren | nach Belieben |

Die Fettkörper werden auf 80 bis 85°C erwärmt. Die wasserlöslichen Bestandteile, darunter die cis-Urocaninsäure, werden bei der gleichen Temperatur in Wasser gelöst, beide Phasen unter kräftigem Rühren miteinander vermischt, und unter mäßigerem Rühren läßt man abkühlen.

### Beispiel 22

| Öl-in-Wasser-Emulsion (Sonnencreme) | |
|---|---|
| UCS | 33,0 g |
| 2,4,6-Trianilino-(p-carbo-2'-ethylhexyl-1'-oxy)-1,3,5-triazin ("Uvinul T-150", BASF) | 18,0 g |
| C₁₂-C₁₅-Alkoholbenzoat ("Finsolv TN", Witco) | 47,0 g |
| Cetylstearylalkohol | 30,0 g |
| Gemisch aus Stearinsäuremono- und diester des Glycerins, sowie Stearinsäureester von Polyethylenoxid ("Arlacel 165", ICI) | 50,0 g |
| Myristylalkohol, polyoxypropyliert mit 3 Mol Propylenoxid ("Witconol APM", Witco) | 185,0 g |
| Wasser | 637,0 g |
| Parfüm, Korrigentien, Additive, Antioxidantien, Stabilisatoren | nach Belieben |

Die Emulsion wird entsprechend vorstehendem Beispiel zubereitet.

### Beispiel 23

| Wasser-in-Öl-Emulsion (Sonnenschutzmilch) | |
|---|---|
| UCS | 20,0 g |
| 1-(4'-tert.Butylphenyl)-3-(4'-methoxyphenyl)-propan-1,3-dion ("Parsol 1789", Givaudan) | 15,0 g |
| 4-Methoxyzimtsäure-2'-ethylhexylester, ("Parsol MCX", Givaudan) | 35,0 g |
| Ester gesättigter Fettsäuren mit Polyethylenoxid ("Arlacel 989", ICI) | 37,0 g |
| Ester ungesättigter Fettsäuren mit Glycerin und Sorbitan ("Arlacel 481", ICI) | 13,0 g |
| Myristylalkohol, polyoxypropoxyliert mit 3 Mol Propylenoxid ("Witconol APM", Witco) | 160,0 g |
| C₁₂-C₁₅-Alkoholbenzoat ("Finsolv TN", Witco) | 40,0 g |
| Magnesiumsulfat-Heptahydrat | 7,0 g |
| Wasser | 673,0 g |
| Parfüm, Korrigentien, Additive, Antioxidantien, Stabilisatoren | nach Belieben |

Die Emulsion wird in der entsprechenden Weise hergestellt wie unter Beispiel 21 beschrieben.

### Beispiel 24

| Wasser-in-Öl-Emulsion (Sonnenschutzmilch) | |
|---|---|
| UCS | 15,0 g |
| 4-Methoxyzimtsäure-2'-ethylhexylester, ("Parsol MCX", Givaudan) | 15,0 g |
| 3-(4'-Methylbenzyliden)campher ("Eusolex 6300", Merck) | 3,0 g |
| Ester ungesättigter Fettsäuren mit Glycerin und ("Arlacel 481", ICI) | 60,0 g |
| Mikrowachs ("Lunacera 11", Fuller) | 10,0 g |
| Capryl-/Caprinsäuretriglycerid ("Miglyol-Neutralöl", Dynamit-Nobel) | 20,0 g |
| Myristylalkohol, polyoxypropyliert mit 3 Mol Propylenoxid ("Witconol APM", Witco) | 145,0 g |
| C₁₂-C₁₅-Alkoholbenzoat ("Finsolv TN", Witco) | 37,0 g |
| Magnesiumstearat | 10,0 g |
| Propylenglykol | 37,0 g |
| Magnesium-Heptahydrat | 7,0 g |
| Wasser | 641,0 g |
| Parfüm, Korrigentien, Additive, Antioxidantien, Stabilisatoren | nach Belieben |

Die Emulsion wird in der entsprechenden Weise hergestellt wie unter Beispiel 21 beschrieben.

### Beispiel 25

| Wasser-in-Öl-Emulsion (Sonnenschutzmilch) | |
|---|---|
| UCS | 33,0 g |
| 4-Methoxyzimtsäure-2'-ethylhexylester ("Parsol MCX", Givaudan) | 15,0 g |
| 3-(4'-Methylbenzyliden)campher ("Eusolex 6300", Merck) | 3,0 g |
| Ester ungesättigter Fettsäuren mit Glycerin und Sorbitan ("Arlacel 481", ICI) | 60,0 g |
| Mikrowachs ("Lunacera 11", Fuller) | 10,0 g |
| Capryl-/Caprinsäuretriglycerid ("Miglyol-Neutralöl", Dynamit-Nobel) | 20,0 g |
| Myristylalkohol, polyoxypropyliert mit 3 mol Propylenoxid ("Witconol APM", Witco) | 119,0 g |
| C₁₂-C₁₅-Alkoholbenzoat ("Finsolv TN", Witco) | 30,0 g |
| Magnesiumstearat | 10,0 g |
| Propylenglykol | 37,0 g |
| Magnesiumsulfat-Heptahydrat | 7,0 g |
| Wasser | 656,0 g |
| Parfüm, Korrigentien, Additive, Antioxidantien, Stabilisatoren | nach Belieben |

Die Emulsion wird in der gleichen Weise hergestellt wie unter Beispiel 21 beschrieben.

### Beispiel 26

| Kationische Emulsion zur Spülung der Haare | |
|---|---|
| UCS | 2,0 g |
| Dimethyldistearylammoniumchlorid ("Arosorf TA 100", Rewo) | 50,0 g |
| Vaseline | 50,0 g |
| Isopropylpalmitat | 20,0 g |
| Cetylalkohol | 10,0 g |
| Wasser | 864,0 g |
| Glycerin | 4,0 g |
| Parfüm, Korrigentien, Additive, Antioxidantien, Stabilisatoren | nach Belieben |

Mit den angegebenen Bestandteilen wird in üblicher Weise eine Haarspülung hergestellt.

Zur Herstellung der kosmetischen Mittel wird die cis-Urocaninsäure in der wäßrigen Phase und der öllösliche UV-Filter in der Fettphase gelöst. In den vorstehenden Beispielen 22, 23, 25, 29 und 30 wird der erfindungsgemäße öllösliche UV-Filter in der Fettphase gelöst und in den Beispielen 24, 26, 27, 28 und 31 die cis-Urocaninsäure in der Wasserphase.

## Patentansprüche

1. Verwendung einer wirksamen Menge an cis-Urocaninsäure als Antioxidans, zur Herstellung von Lichtschutzformulierungen für kosmetische und/oder dermatologische Zwecke.

2. Verwendung eines Gemisches von cis- und trans-Urocaninsäure als Antioxidans in kosmetischen und/oder dermatologischen Formulierungen, wobei gewährleistet sein muß, daß wenigstens die cis-Urocaninsäure in einer wirksamen Konzentration vorliegt.

3. Verwendung nach Anspruch 1 oder 2, dadurch gekennzeichnet, daß in den kosmetischen und/oder dermatologischen Formulierungen 0,01 bis 10 Gew.-% an cis-Urocaninsäure, vorzugsweise 0,1 - 6,0 Gew.-%, bezogen auf das Gesamtgewicht der Formulierung, enthalten sind.

4. Verwendung nach Anspruch 1 oder 2, dadurch gekennzeichnet, daß die kosmetischen und/oder dermatologischen Formulierungen zusätzlich mindestens einen UVB-Filter und/oder mindestens einen weiteren UVA-Filter und/oder mindestens ein anorganisches Pigment enthält.

5. Verwendung nach Anspruch 1 oder 2, dadurch gekennzeichnet, daß die kosmetischen und/oder dermatologischen Formulierungen Pigmente auf der Basis von Titandioxid enthalten.

## Claims

1. Use of an active amount of cis-urocaninic acid as an antioxidant, for the preparation of light protection formulations for cosmetic and/or dermatological purposes.

2. Use of a mixture of cis- and trans-urocaninic acid as an antioxidant in cosmetic and/or dermatological formulations, wherein it must be ensured that at least the cis-urocaninic acid is present in an active concentration.

3. Use according to Claim 1 or 2, characterized in that the cosmetic and/or dermatological formulations comprise 0.01 to 10% by weight of cis-urocaninic acid, preferably 0.1 - 6.0% by weight, based on the total weight of the formulation.

4. Use according to Claim 1 or 2, characterized in that the cosmetic and/or dermatological formulations additionally comprises [sic] at least one UVB filter and/or at least one other UVA filter and/or at least one inorganic pigment.

5. Use according to Claim 1 or 2, characterized in that the cosmetic and/or dermatological formulations comprise pigments based on titanium dioxide.

## Revendications

1. Utilisation d'une quantité efficace d'acide *cis*-urocanique en tant qu'antioxydant pour la préparation de formulations photoprotectrices à des fins cosmétiques et/ou dermatologiques.

2. Utilisation d'un mélange d'acide *cis*- et d'acide trans-urocanique en tant ou' antioxydant dans des formulations cosmétiques et/ou dermatologiques, dans lesquelles il doit être garanti qu'au moins l'acide *cis*-urocanique est présent à une concentration efficace.

3. Utilisation selon la revendication 1 ou 2, caractérisée en ce que de 0,01 à 10 % en poids, de préférence de 0,1 à 6,0 % en poids, d'acide *cis*-urocanique, par rapport au poids total de la formulation, sont contenus dans les formulations cosmétiques et/ou dermatologiques.

4. Utilisation selon la revendication 1 ou 2, caractérisée en ce que les formulations cosmétiques et/ou dermatologiques contiennent en outre au moins un filtre UVB et/ou au moins un autre filtre UVA et/ou au moins un pigment minéral.

5. Utilisation selon la revendication 1 ou 2, caractérisée en ce que les formulations cosmétiques et/ou dermatologiques contiennent des pigments à base de dioxyde de titane.
